# EUROPEAN PATENT APPLICATION

(11) **EP 1 676 580 A1**
(43) Date of publication of application: **05.07.2006**
(21) Application number: 04771449.8
(22) Date of filing: 10.08.2004
(51) Int. Cl.: A61K 31/704, A61K 31/198, A61P 1/16, A61P 37/08

(54) **GLYCYRRHIZIN HIGH-CONCENTRATION PREPARATION**

(30) Priority: 12.08.2003 JP 2003292135
(71) Applicant: NIPPON ZOKI PHARMACEUTICAL CO. LTD., Osaka-shi, Osaka 541-0046 (JP)
(72) Inventor: YOSHIKAWA, Taro, NIPPON ZOKI PHARMACEUTICAL CO LTD, Hirano-ku, Osaka-shi, Osaka 5470044 (JP); HANAOKA, Satoshi NIPPON ZOKI PHARMACEUTICAL COLTD, Hirano-ku, Osaka-shi, Osaka 5470044 (JP)
(74) Representative: HOFFMANN EITLE
(86) International application number: PCT/JP2004/011462
(87) International publication number: WO 2005/014009

(57) **Abstract**

[PROBLEMS] To provide a glycyrrhizin/aminoacetic acid/cysteine combination drug containing an active ingredient in high concentration and excelling in stability and safety. [MEANS FOR SOLVING PROBLEMS] Stability at compounding of active ingredient in high concentration has been improved by avoiding the addition of sulfite salts having been used as a stabilizer in existing products. As glycyrrhizin high-concentration preparations, there is provided a drug composition comprising 8 to 16 mg/mL of glycyrrhizin, 3 to 6 mg/mL of cysteine and 80 to 160 mg/mL of aminoacetic acid wherein no sulfite salts are added as an additive.

## Description

### Technical Field

The present invention relates to a pharmaceutical composition containing high concentrations of glycyrrhizin, cysteine and aminoacetic acid (glycine) which are useful as drugs for hepatic diseases or for allergy.

### Background Art

It has been known already that glycyrrhizins have various kinds of pharmacological actions such as anti-cortisone action, decholesterolizing action, anti-allergic action, anti-inflammatory action, detoxifying actin and reparative action for gastric ulcer and their safety has been also confirmed whereby glycyrrhizin preparations containing the same as an effective ingredient have been widely used as remedies for various diseases. In recent years, efficacy of a megadose of glycyrrhizin by intravenous injection to chronic hepatic diseases has been reported whereby utility of glycyrrhizin preparations has been reconsidered.

In general, it is often in the case of drugs for the treatment of hepatic disease that drug is continuously administered for a relatively long period. A combination drug of glycyrrhizin, aminoacetic acid and cysteine which has now been available in the market (trade name: Stronger Neo-Minophagen C) is an injection preparation in which 2.65 mg/mL of monoammonium glycyrrhizinate (2 mg/mL as glycyrrhizin), 1 mg/mL of cysteine hydrochloride (0.77 mg/mL as cysteine) and 20 mg/mL of aminoacetic acid are compounded. Although its dose is appropriately increased or decreased depending upon age and symptom, it is intravenously injected or intravenously dripped at the dose of 40 to 60 mL a day once daily (which may be increased up to 100 mL) to chronic hepatic diseases. Such an administration in large dose results in a problem that not only pain to a patient is resulted upon administration but also administration of day after day for long time makes the tissue of the injected site thick. There are other problems that glycyrrhizin which is a component compounded in this preparation is precipitated or that cysteine hydrochloride is apt to be degraded and is unstable (refer to Patent Document 1) and, in the existing injection preparations, a sulfite (0.8 mg/mL of sodium sulfite) is added as a stabilizer (refer to Non-Patent Document 1). However, there is a report that a sulfite is an attractant for onset of asthma (refer to Non-Patent Document 2) and it has also been reported as a food additive which induces allergy (refer Non-Patent Documents 3 and 4). Accordingly, pharmaceutical preparations containing them in high concentrations have a problem in terms of safety.

Patent Document 1: Japanese Patent Laid-Open No. 2002/065,808, page 2, column 0004

Non-Patent Document 1: Package Insert for a Drug "Stronger Neo-Minophagen C" (prepared by K. K. Minophagen Seiyaku)

Non-Patent Document 2: Tatsuo Sakamoto: "Food Additives and Asthma (Mainly Concerning Sulfites), Airway Allergy" '96, Medical View Firm; page 151, 1996

Non-Patent Document 3: Yoichi Kawano: Fundamentals and Clinics of Food Allergy (Allergenicity of Food), Allergology & Immunology, 4(6), pages 741 to 745, 1997

Non-Patent Document 4: Masataka Michibata: Self-Control of Steroids, My means, Climics & Drug Therapy, 16 (3), pages 226 to 230, 1997

### Disclosure of the Invention

### Problems that the Invention is to Solve

As mentioned above, in administration ofa combination drug of glycyrrhizin, aminoacetic acid and cysteine, there has been a demand for making the burden of patents as little as possible such as pain and tissue thickening of the injected site upon megadose by intravenous injection and the likewhereby the present inventors have carried out intensive studies for high-concentration preparations where a pharmaceutical effect can be expected by administration of small dose and where stability and safety are high.

An object of the present invention is to provide a combination drug of glycyrrhizin, aminoacetic acid and cysteine where effective ingredients are compounded in higher concentrations than the conventional product and also has high stability and safety. Even when concentrations of the compounded components in the conventional product are merely made high, degradation, precipitation and the like of effective ingredients are resulted and no sufficient stability is available. Further, problem in terms of safety by the sulfite contained therein is also resulted. Means for Solving the Problems

In order to solve the aforementioned problem, the present inventors have carried out intensive studies and found that, when sodium sulfite which has been used as a stabilizer in the conventional product is not used, stability when effective ingredients are compounded in high concentrations are improved and a combination drug of glycyrrhizin, aminoacetic acid and cysteine are compounded in which effective ingredients are contained in higher concentrations than the conventional product and are with high safety is able to be prepared whereupon the present invention has been achieved.

### Advantages of the Invention

In the pharmaceutical composition of the present invention, a sulfite which has been used as a stabilizer in the conventional product is not used and, as a result, precipitate of glycyrrhizin which is compounded in a high concentration is not produced, reduction in the amount of cysteine therein is low and stability is enhanced.

### Best Mode for Carrying Out the Invention

The present invention relates, in a glycyrrhizin preparation where glycyrrhizin or a pharmacologically acceptable salt is an effective ingredient, to a combination drug of glycyrrhizin, aminoacetic acid and cysteine in high concentrations which is characterized in that cysteine and aminoacetic acid are contained in addition to the aforementioned ingredient and that no sulfite is contained therein.

Glycyrrhizin which is an effective ingredient of the pharmaceutical composition of the present invention is able to be prepared by extracting from licorice root or that which has been available in the market may be used as well. Glycyrrhizin is also called glycyrrhizinic acid and, in the present invention, it includes a pharmaceutically acceptable salt of glycyrrhizin. Examples of the pharmaceutically acceptable salt are salts with acid or base including ammonium salt such as monoammonium glycyrrhizinate and alkali metal salt such as disodium glycyrrhizinate, trisodium glycyrrhizinate and dipotassium glycyrrhizinate.

Further, cysteine and aminoacetic acid (glycine) in the present invention also include pharmaceutically acceptable salts thereof and their examples cover both kinds of salts of an acid addition salt such as that with hydrochloric acid or malic acid and a base addition salt such as that with alkali metal (e.g., sodium), alkali earth metal, ammonium and nitrogen-containing organic base. A preferred salt of cysteine is a hydrochloride. Moreover, a hydrate of cysteine, aminoacetic acid or a salt thereof such as cysteine hydrochloride monohydrate and sodium aminoacetate hydrate may be also included as cysteine and aminoacetic acid of the present invention. There are optically active substances in cysteine and any of L- and racemic substances may be used and, preferably, L-cysteine may be used.

The preferred compounding amounts in the pharmaceutical composition of the present invention are in amounts of from 4- to 8-fold of those of the effective ingredients in the aforementioned conventional preparation. Thus, it is a pharmaceutical composition containing 8 to 16 mg/mL of glycyrrhizin, 4 to 8 mg/mL of cysteine hydrochloride (3 to 6 mg/mL as cysteine) and 80 to 160 mg/mL of aminoacetic acid. Particularly preferably, it is a pharmaceutical composition where effective ingredients are contained in the highest concentrations or containing 16 mg/mL of glycyrrhizin, 8 mg/mL of cysteine hydrochloride and 160 mg/mL of aminoacetic acid. Incidentally, each of the aforementioned values for the concentrations (mg/mL) is in accordance with the regulation for standard values mentioned in General Rule 18 of the Japanese Pharmacopoeia (the 14th revision) and is a value rounding off the first decimal place.

The pharmaceutical composition of the present invention may also be made into the final drug by combining with an appropriate pharmaceutical carrier or diluent and may be made into pharmaceutical preparations by any of common methods. For example, with regard to an injection preparation, it may be made into a solution or a suspension of an aqueous solvent or a non-aqueous solvent, such as distilled water for injection, physiological saline solution, Ringer's solution, vegetable oil, synthetic fatty acid glyceride, higher fatty acid ester and propylene glycol. In formulating the preparation, it may be made into a combination drug with other pharmaceutically active ingredient.

### Example 1

Influence by addition of sodium sulfite (1)

Monoammonium glycyrrhizinate, cysteine hydrochloride and aminoacetic acid were dissolved in water where the oxygen dissolved therein was small so as to make their compounding ratio 16 mg/mL (as glycyrrhizin), 8 mg/mL and 160 mg/mL, respectively. The solution was adjusted to pH from 7.2 to 7.5 with sodium hydroxide. After sodium sulfite was added thereto as a stabilizer so as to make 0, 2.4 or 4.0 mg/mL, the dissolved oxygen was removed using nitrogen. The solution was filtered, sterilized and filled in ampoules together with nitrogen. The ampoules were stored at 25°C for four years and the state of precipitation of glycyrrhizin was observed. Separately, the ampoules were stored at 40°C for four months or at 60°C for 14 days and cysteine contained therein was quantified by an HPLC. In this manner, stability due to the difference in the adding amount of sodium sulfite in the pharmaceutical composition of the present invention was measured. An example of results is shown in Table 1.

**Table 1**

| | | | Adding amount of sodium sulfite (mg/mL) | | |
|---|---|---|---|---|---|
| | | | 0 | 2.4 | 4.0 |
| pH at manufacturing | | | 7.22 | 7.49 | 7.29 |
| Presence or absence of precipitation of glycyrrhizin | After 4 years at 25°C | | - | + | + |
| Amount of cysteine hydrochloride(%) | Before sterilization | | 97.3 | 101.6 | 98.9 |
| | After sterilization | | 94.4 | 95.2 | 91.1 |
| | 60°C | After 3 days | 89.7 | 87.8 | 81.4 |
| | | After 7 days | 81.2 | 71.3 | 64.2 |
| | | After 14 days | 77.8 | 66.5 | 53.9 |
| | 40°C | After 2 months | 89.4 | 86.0 | 70.3 |
| | | After 4 months | 83.6 | 77.7 | 68.5 |

### Example 2

### Influence by addition of sodium sulfite (2)

In the same manner as in the above Example 1, monoammonium glycyrrhizinate, cysteine hydrochloride and aminoacetic acid were dissolved in water where the oxygen dissolved therein was small so as to make their compounding ratio 16 mg/mL (as glycyrrhizin), 8 mg/mL and 160 mg/mL, respectively. The solution was adjusted to pH 7.2 to 7.5 with sodium hydroxide. Then there were prepared a product to which 6.4 mg/mL of sodium sulfite as a stabilizer was added and that to which no sodium sulfite was added. Each of them was also dilutedinto preparations so as to make concentrations of cysteine hydrochloride 6 mg/mL and 4 mg/mL. Oxygen dissolved therein was removed from them and the solution was filtered, sterilized and filled in ampoules with nitrogen. The ampoules were stored at 60°C for 14 days and, at the stages of initiation and after 4 days, 7 days and 14 days, cysteine contained therein was quantified by an HPLC. In this manner, stability due to the difference in the adding amount of sodium sulfite in the pharmaceutical composition of the present invention was measured. An example of results is shown in Table 2.

**Table 2**

| Concentration of cysteine hydrochloride (mg/mL) | | 8 | 6 | 4 |
|---|---|---|---|---|
| Non-addition | Initial | 100.0 | 100.0 | 100.0 |
| | 4 days | 93.5 | 97.5 | 94.3 |
| | 7 days | 88.6 | 94.5 | 89.5 |
| | 14 days | 82.8 | 82.4 | 88.7 |
| Addition of sodium sulfite | Initial | 100.0 | 100.0 | 100.0 |
| | 4 days | 73.6 | 84.4 | 88.8 |
| | 7 days | 53.1 | 70.4 | 77.1 |
| | 14 days | 40.4 | 52.2 | 59.8 |

### Industrial Applicability

As apparent from the result shown in the aforementioned Tables 1 and 2, the higher the sodium sulfite concentration is, the more the reduction in the amount of cysteine with lapse of time is. In the cases where sodium sulfite was added, precipitate of glycyrrhizin was produced while, in the cases where no sodium sulfite was added, not only precipitate of glycyrrhizin which was added in a high concentration was not produced but also reduction in the amount of cysteine was low whereby stability was improved. As such, in the preparation of the present invention where glycyrrhizin was concentrated, amounts of effective ingredients are in higher concentrations than in the conventional preparations and both stability and safety are also excellent whereby its utility as pharmaceuticals is very high.

## Claims

1. A pharmaceutical composition containing 8 to 16 mg/mL of glycyrrhizin, 3 to 6 mg/mL of cysteine and 80 to 160 mg/mL of aminoacetic acid.

2. The pharmaceutical composition according to claim 1, wherein no sulfite is contained.

3. The pharmaceutical composition according to claim 1 or 2, wherein glycyrrhizin is monoammonium glycyrrhizinate.

4. The pharmaceutical composition according to any one of claims 1 to 3, wherein cysteine is cysteine hydrochloride.

5. A pharmaceutical composition containing 16 mg/mL of glycyrrhizin, 8 mg/mL of cysteine hydrochloride and 160 mg/mL of aminoacetic acid and containing no sulfite as an additive.
